# EUROPÄISCHE PATENTSCHRIFT

(11) **EP 0 804 138 B1**
(45) Veröffentlichungstag und Bekanntmachung des Hinweises auf die Patenterteilung: **31.03.1999**
(21) Anmeldenummer: 96900914.1
(22) Anmeldetag: 08.01.1996
(51) Int. Cl.: A61K 7/00

(54) **2-PHASEN-HAARBEHANDLUNGSMITTEL**
2-PHASE HAIR TREATMENT MEDIUM
AGENT DE SOINS CAPILLAIRES A DEUX PHASES

(30) Priorität: 17.01.1995 DE 19501187
(43) Veröffentlichungstag der Anmeldung: 05.11.1997
(73) Patentinhaber: Henkel Kommanditgesellschaft auf Aktien, 40191 Düsseldorf (DE)
(72) Erfinder: HOLLENBERG, Detlef, D-40699 Erkrath (DE); SEIDEL, Kurt, D-40589 Düsseldorf (DE); PRIEBE, Christian, D-42489 Wülfrath (DE)
(86) Internationale Anmeldenummer: EP9600041
(87) Internationale Veröffentlichungsnummer: WO9622071

(56) Entgegenhaltungen:
- WO-A-92/13829
- DE-C- 4 100 490

## Beschreibung

Die Erfindung betrifft Mittel, insbesondere kosmetische Mittel, in Form von 2-Phasen-Zubereitungen.

Das menschliche Haupthaar wird heute in vielfältiger Weise mit haarkosmetischen Zubereitungen behandelt. Dazu gehört beispielsweise die Reinigung der Haare mit Shampoos und Duschpräparaten und das Bleichen, Färben und Verformen von Haaren mit Wellmitteln, Tönungsmitteln und Stylingpräparaten. Als Folge von Haarbehandlungen kann es zu unerwünschten Beeinträchtigungen der Haarstruktur kommen. Diese Beeinträchtigungen zeigen sich u.a. in einer schlechten Naß- und Trockenkämmbarkeit, einer verstärkten elektrostatischen Aufladung, verstärkter Sprödigkeit sowie in gravierenderen Fällen in gesplißten, d.h. aufgespaltenen Haar(end)en. Dies verschlechtert nicht nur das äußere Erscheinungsbild der Frisur, sondern insbesondere dem Haarspliß sollte auch aufgrund der Haargesundheit entgegengewirkt werden.

Es wurde daher eine Reihe von Produkten entwickelt, die eine oder mehrere der folgenden Aufgaben erfüllen:
- Abbau der durch eine Haarbehandlung verursachten Schädigungen durch Regeneration des Haares,
- Vermeidung oder Entgegenwirkung der durch Haarschädigungen hervorgerufenen negativen Auswirkungen auf das äußere Erscheinungsbild der Frisur und die Handhabbarkeit der Haare sowie
- Prävention möglicher, durch Umwelteinflüsse oder Haarbehandlungen hervorgerufener, Haarschädigungen durch gezielte Zufuhr von Wirkstoffen.

Eine Reihe solcher Produkte sind unter Bezeichnungen wie "Haarnachbehandlungsmittel", "Pflegespülung", "Haarwasser", "Haarkur", "Haarbalsam" etc. auf dem Markt. Diese Mittel werden in der überwiegenden Zahl der Fälle als Emulsionen oder wäßrige Lösungen formuliert. Eine solche Darreichungsform ist aber nur dann möglich, wenn es die zu verwendenden Wirkstoffe erlauben, eine den Verbraucher auch optisch ansprechende Formulierung herzustellen. Gerade bei Produkten, die explizit zur Gesunderhaltung und Verschönerung des Erscheinungsbildes des Haares ausgelobt werden, ist der Konsument äußerst kritisch gegenüber Produkten, die aufgrund von Ausfällungen, unschön erscheinenden Emulsionen etc. der mental assoziierten "Pflegeerwartung" zu widersprechen scheinen.

Es ist bekannt, daß durch 2-Phasen-Zubereitungen, die erst kurz vor der Anwendung durch Schütteln in eine kurzfristig stabile Emulsion überführt werden, einige dieser Schwierigkeiten überwunden werden können .

Im Kosmetikbereich sind solche Zubereitungen beispielsweise aus den Druckschriften DE-OS-16 17 808, DE-C1-36 27 313, DE-A1-29 05 257, DE-A1-31 10 258, DE-C1-41 00 490 und DE-C1 42 41 799 bekannt. In Mitteln gemäß dieser Druckschriften werden 2-Phasen-Zubereitungen insbesondere deshalb verwendet, um öllösliche Wirkstoffe in Form einer, vom Verbraucher gefühlsmäßig bevorzugten, Emulsion auftragen zu können. Die Herstellung in Form einer stabilen Emulsion ist aber nicht oder nur unter Schwierigkeiten möglich, auf entsprechende Emulgatormengen oder Stabilisierungsmittel soll aus anderen Erwägungen verzichtet werden oder die Emulsionen haben sich bei der Anwendung auf Haut oder Haar als weniger geeignet erwiesen.

Die Druckschriften befassen sich aber mit keinem Wort mit weiteren Problemen wie z.B.
- Inkompatibilität von öllöslichen mit wasserlöslichen Wirkstoffen und
- Feuchtigkeitsempfindlichkeit von öllöslichen Wirkstoffen.

Es wurde nun überraschenderweise gefunden, daß u.a. auch diese Probleme mit speziellen 2-Phasen-Zubereitungen überwunden werden können. So sind beispielsweise feuchtigkeitsempfindliche Wirkstoffe im Rahmen einer Emulsion applizierbar, wenn durch geeignete Emulgatoren für ein sehr schnelles Aufbauen der Emulsion gesorgt wird, und, im Falle mehrfacher Anwendung aus einem Vorratsgefäß, die Emulsion hinreichend schnell wieder bricht. Weiterhin können analog öllösliche und wasserlösliche Wirkstoffe, die in Emulsionen inkompatibel sind, zusammen in einem Mittel appliziert und in Form einer Emulsion auf das Substrat aufgebracht werden.

Gegenstand der Erfindung ist somit eine in Form eines 2-Phasen-Systems vorliegende, durch mechanische Einwirkung kurzzeitig mischbare Zubereitung zur Behandlung keratinischer Gebilde, insbesondere menschlicher Haare und menschlicher Haut, dadurch gekennzeichnet, daß sie
- ein kationisch derivatisiertes Panthenol (A) der allgemeinen Formel (I), 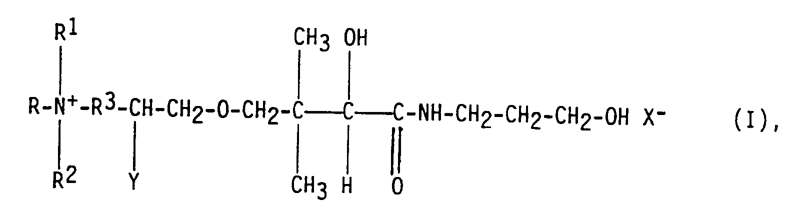 in der R, R¹ und R² unabhängig voneinander für eine Alkylgruppe mit 1-24 Kohlenstoffatomen oder eine Alkenylgruppe mit 8-24 Kohlenstoffatomen, R³ für eine Alkylengruppe mit 1-18 Kohlenstoffatomen, Y für eine OH-Gruppe oder für Wasserstoff und X für ein einwertiges, organisches oder anorganisches Anion steht und/oder
- einen Polyoxyalkylen-Ether eines Polyglycerin-Fettsäureesters (B)
enthält.

Wenngleich solche Zubereitungen bevorzugt im Kosmetikbereich angewendet werden, so stellt dies jedoch keine Einschränkung dar.

Erfindungsgemäß ist der Begriff "kurzzeitig mischbar" durch folgenden Test definiert:
Die 2-Phasen-Zubereitung wird in ein Reagenzglas mit ca. 1 cm Durchmesser in einer solchen Menge gebracht, daß mindestens 90 % des Volumens gefüllt sind. Das verschlossene Reagenzglas wird jeweils durch Drehen um die kurze Achse um 180° 5 mal innerhalb von 5 Sekunden "auf den Kopf gestellt" und wieder in die Ausgangslage gebracht. Dann wird das Reagenzglas ruhig in der ursprünglichen Lage belassen. Eine 2-Phasen-Zubereitung gilt als "kurzfristig mischbar" im Sinne der Erfindung, wenn bei Betrachtung mit dem bloßen Auge folgende 3 Bedingungen erfüllt sind:
1. Durch die Bewegung bildet sich ein 1-Phasen-System,
2. 5 - 30 Sekunden nach Ende der Bewegung ist das Auftreten einer zweiten Phase erkennbar,
3. nach spätestens 30 Stunden ist die Phasentrennung vollständig abgeschlossen.

Dabei haben sich solche Systeme als anwendungstechnisch besonders geeignet erwiesen, bei denen die Abtrennung der 2. Phase (Bedingung 2) nach 10-20 Sekunden begann und nach 24 Stunden beendet ist (Bedingung 3).

Bei den beiden Phasen kann es sich um eine rein wäßrige Phase, gegebenenfalls mit weiteren gelösten Bestandteilen und um eine reine Öl-Phase, ebenfalls gegebenenfalls mit weiteren gelösten Bestandteilen, handeln.

Üblicherweise wird aber die Wasserphase auch Ölanteile und die Ölphase auch Wasseranteile enthalten. Es ist bevorzugt, daß die wäßrige Phase entweder keine, oder nur geringe Ölanteile enthält. Eine solche Emulsion hat dann nicht mehr als 5 Gew.-% Ölanteile, bezogen auf die Komponenten Wasser und Öl.

Als Öl-Phase wird in der Regel eine Emulsion vorliegen, die bis zu 20 Gew.-% Wasser, bezogen auf die Komponenten Öl und Wasser, enthält.

Die erfindungsgemäßen Zubereitungen enthalten die beiden Phasen bevorzugt in einem Verhältnis von 1:1 bis 5:1. Massenverhältnisse von 2:1 bis 1:2 von Öl-Phase und Wasserphase sind dabei besonders bevorzugt.

Öle im Sinne der Erfindung sind mineralische Öle, pflanzliche Öle und synthetische Öle, wie beispielsweise Silikonöle. Wegen ihrer besonderen anwendungstechnischen Eigenschaften kann es bevorzugt sein, als Ölphase Paraffine oder Silikonöle, insbesondere Polydimethylsiloxane niedriger Viskositäten, einzusetzen. Ein entsprechendes Silikonöl ist beispielsweise unter der Bezeichnung Dow Corning^{R}200 Fluid im Handel.

Solche Systeme haben besonders vorteilhafte Eigenschaften, wenn sie einen Emulgator von einem bestimmten kationischen oder einem bestimmten nichtionischen Typ enthalten.

Die Emulgatoren vom kationischen Typ sind Derivate des Panthenols gemäß Formel (I). Solche Verbindungen sind beispielsweise aus der PCT-Offenlegungsschrift WO 92/13829 bekannt als kosmetische Wirkstoffe, insbesondere für Haarbehandlungsmittel.

Bevorzugt sind dabei solche Verbindungen gemäß Formel (I), bei denen R für eine Alkyl- oder Alkenylgruppe mit 8-24 Kohlenstoffatomen steht. Solche Alkyl- bzw. Alkenylgruppen sind beispielsweise Lauryl-, Myristyl-, Cetyl-, Stearyl- und Oleyl-Gruppen. R¹ und R² sind bevorzugt Alkylgruppen mit 1-4 Kohlenstoffatomen, insbesondere Methylgruppen. R³ ist ebenfalls bevorzugt eine kürzere Alkylengruppe mit 1-4 Kohlenstoffatomen, insbesondere eine Methylengruppe. Y steht, herstellungsbedingt, bevorzugt für eine Hydroxygruppe, X für ein Halogenidion, insbesondere für Chlorid.

Informationen bezüglich der Herstellung dieser Panthenol-Derivate sind der genannten Druckschrift WO 92/13829 zu entnehmen, auf die hier ausdrücklich Bezug genommen wird. Ein entsprechendes Produkt wird von der Firma Tri-K unter der Bezeichnung PANTHEQUAT^{R} vertrieben.

Die Emulgatoren vom nichtionischen Typ sind spezielle Polyglycerin-Fettsäure-Ester. Bevorzugte Emulgatoren (B) enthalten
- 2 bis 20 Oxyalkylen-, insbesondere Oxyethylen-, Einheiten,
- 2 bis 10 Glycerin-Einheiten sowie
- 1 oder 2 Fettsäureeinheiten mit 8 bis 24 Kohlenstoffatomen.

Verbindungen mit bis zu 5 Glycerin-Einheiten, Oxyethylen-Einheiten und einer Fettsäureeinheit haben sich als erfindungsgemäß besonders geeignet erwiesen. Die Fettsäureeinheiten können sowohl gesättigt als auch ungesättigt sein; Verbindungen auf Basis Laurin-, Myristin-, Palmitin-, Stearin- und Öl-Säure haben sich als besonders geeignet erwiesen.

Emulgatoren vom Typ (B) sind im Handel erhältlich; so vertreibt die Hoechst AG unter der Bezeichnung Hostacerin^{R}-DGL einen Poly(10)ethylenglykolether eines mit Laurinsäure veresterten Diglycerins.

Die kationisch derivatisierten Panthenole sind in den erfindungsgemäßen Zubereitungen bevorzugt in einer Menge von 0,05 bis 1 Gew.-%, insbesondere 0,05 bis 0,2 Gew-%, bezogen auf die gesamte Zubereitung, enthalten.

Die Emulgatoren vom Typ (B) sind in den erfindungsgemäßen Zubereitungen bevorzugt in einer Menge von 0,05-1 Gew.-%, insbesondere 0,1-0,5 Gew-%, bezogen auf die gesamte Zubereitung, enthalten.

Entsprechend der Aufgabe der erfindungsgemäßen Zubereitung enthält diese zumindest einen kosmetischen Wirkstoff, sowie übliche Hilfs- und Zusatzstoffe. In der Regel unterliegen die erfindungsgemäßen Zubereitungen hinsichtlich dieser Bestandteile keinen Beschränkungen. Lediglich bei weiteren Tensiden, die das Mischungsverhalten der beiden Phasen und die Emulsionsstabilität stark beeinflussen können, muß der Fachmann die Eignung mit Hilfe des oben genannten einfachen Testes im Zweifelsfalle überprüfen; gleiches gilt für den Fall, daß große Mengen an viskositätssteigernden Mitteln eingesetzt werden sollen.

Übliche Wirk-, Hilfs- und Zusatzstoffe für die erfindungsgemäßen Zubereitungen sind beispielsweise
- anionische Tenside, wie beispielsweise Fettalkylsulfate- und -ethersulfate sowie Alkylethercarbonsäuren,
- zwitterionische Tenside, wie beispielsweise Betaine,
- ampholytische Tenside,
- nichtionogene Tenside, wie beispielsweise Alkylpolyglycoside und ethoxylierte Fettalkohole,
- kationische Tenside, wie beispielsweise quartäre Ammoniumverbindungen, Amidoamine und sogenannte "Esterquats",
- nichtionische Polymere wie beispielsweise Vinylpyrrolidon/Vinylacrylat-Copolymere, Polyvinylpyrrolidon und Vinylpyrrolidon/Vinylacetat-Copolymere und Polysiloxane,
- kationische Polymere wie quaternisierte Celluloseether, Polysiloxane mit quaternären Gruppen, Dimethyldiallylammoniumchlorid-Polymere, Acrylamid-Dimethyldiallylammoniumchlorid-Copolymere, mit Diethylsulfat quaternierte Dimethylaminoethylmethacrylat-Vinylpyrrolidon-Copolymere, Vinylpyrrolidon-Imidazoliniummethochlorid-Copolymere und quaternierter Polyvinylalkohol,
- zwitterionische und amphotere Polymere wie beispielsweise Acrylamidopropyl-trimethylammoniumchlorid/Acrylat-Copolymere und Octylacrylamid/Methyl-methacrylat/tert.Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Maleinsäureanhydrid-Copolymere und Acrylsäure/Ethylacrylat/N-tert.Butylacrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Wirkstoffe wie Panthenol, Allantoin, Pyrrolidoncarbonsäuren und deren Salze, Pflanzenextrakte und Vitamine,
- Lichtschutzmittel,
- Strukturanten wie Glucose und Maleinsäure,
- haarkonditionierende Verbindungen wie Phospholipide, beispielsweise Sojalecithin, Ei-Lecithin und Kephaline,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes,
- Konsistenzgeber wie Zuckerester, Polyolester oder Polyolalkylether,
- Fette und Wachse, wie Walrat, Bienenwachs, Montanwachs und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Antioxidantien,
- direktziehende Farbstoffe,
- sogenannte Kuppler- und Entwicklerkomponenten als Oxidationsfarbstoffvorprodukte,
- Reduktionsmittel wie z.B. Thioglykolsäure und deren Derivate, Thiomilchsäure, Cysteamin, Thioäpfelsäure und α-Mercaptoethansulfonsäure,
- Oxidationsmittel wie Wasserstoffperoxid, Kaliumbromat und Natriumbromat.

Die erfindungsgemäßen Zubereitungen unterliegen hinsichtlich der Art des Mittels keinerlei weiteren Beschränkungen. Bevorzugt sind aber kosmetische Mittel, insbesondere Haarbehandlungsmittel. Innerhalb der Gruppe der Haarbehandlungsmittel sind die erfinderischen Zubereitungen insbesondere geeignet für Haarpflegeprodukte wie Haarkuren, Haarbalsame etc., die ohne weiteres Spülen auf dem Haar verbleiben. Gegenstand der Erfindung ist daher ebenfalls ein Verfahren zur Behandlung von Haaren, bei dem eine erfindungsgemäße Zubereitung auf das Haar aufgebracht wird und dort verbleibt.

Es können aber auch Produkte formuliert werden, die nach einer gewissen Einwirkzeit (üblicherweise ca. 30 Sekunden bis ca. 30 Minuten) wieder aus dem Haar ausgespült werden. Gegenstand der Erfindung ist daher auch ein Verfahren zur Behandlung von Haaren, bei dem eine erfindungsgemäße Zubereitung auf das Haar aufgebracht und nach einer Einwirkzeit wieder ausgespült wird.

Die folgenden Beispiele sollen die Erfindung näher erläutern.

### Beispiele

### Formulierungsbeispiele:

### Alle Angaben sind in Gewichts-%.

| 1. Haarkur: | |
|---|---|
| Ölphase: | |
| Dow Corning^{R}345¹ | 29,0 |
| Cetiol^{R}OE² | 1,0 |

| Wasserphase: | |
|---|---|
| Wasser | 68,55 |
| Pathequat^{R} ³ | 0,4 |
| Luviskol^{R} K 30⁴ | 0,2 |
| Mirapol^{R}A 15⁵ | 0,8 |
| Euxyl^{R} K 400⁶ | 0,05 |

| | |
|---|---|
| ¹ Decamethylpentasiloxan (CTFA-Bezeichnung: Cyclomethicone) (DOW CORNING) | |
| ² Dioctylether (CTFA-Bezeichnung: Dicaprylether) (HENKEL) | |
| ³ kationisches Panthenolderivat (45 % Aktivsubstanz; CTFA-Bezeichnung: Panthenyl Hydroxypropylsteardimoniumchlorid) (TRI-K Industries) | |
| ⁴ Polyvinylpyrrolidon (95 % Aktivsubstanz, Rest Wasser) (BASF). | |
| ⁵ Poly-[N-(3-dimethylammonium)propyl-N'-(3-ethylenoxyethylen-dimethylammonium)propyl-harnstoff]-dichlorid (64 % Aktivsubstanz in Wasser; CTFA-Bezeichnung: Polyquaternium 2) (MIRANOL) | |
| ⁶ 1,2-Dibrom-2,4-dicyanobutan-Phenoxyethanol-Gemisch (SCHÜLKE & MAYR) | |

| 2. Haarkur: | |
|---|---|
| Ölphase: | |
| Dow Corning^{R}345 | 38,0 |
| Diisopropyladipat | 1,5 |
| Parfumöl | 0,5 |

| Wasserphase: | |
|---|---|
| Wasser | 58,7 |
| Hostacerin^{R}DGL⁷ | 0,6 |
| Glucose | 0,1 |
| Lamequat^{R}L⁸ | 0,5 |
| p-Hydroxybenzoesäuremethylester | 0,1 |

| | |
|---|---|
| ⁷ Fettsäurepolyglycerinester-Oxethylat (CTFA-Bezeichnung: PEG-10 polyglyceryl-2-laurate) (HOECHST) | |
| ⁸ kationisiertes Kollagenhydrolysat (35 % Aktivsubstanz in Wasser; CTFA-Bezeichnung: Lauryldimonium Hydroxypropyl Hydrolyzed Collagen) (HENKEL) | |

## Patentansprüche

1. In Form eines 2-Phasen-Systems vorliegende, durch mechanische Einwirkung kurzzeitig mischbare Zubereitung zur Behandlung keratinischer Gebilde, insbesondere menschlicher Haare und menschlicher Haut, dadurch gekennzeichnet, daß sie
- ein kationisch derivatisiertes Panthenol (A) der allgemeinen Formel (I), 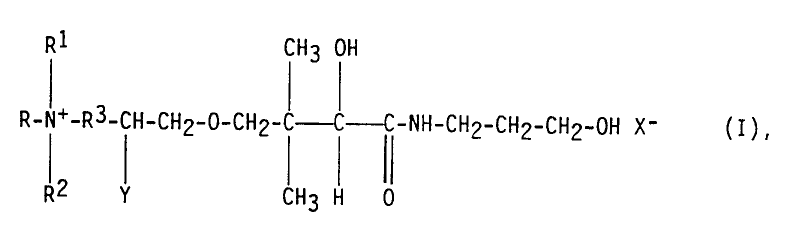 in der R, R¹ und R² unabhängig voneinander für eine Alkylgruppe mit 1-24 Kohlenstoffatomen oder eine Alkenylgruppe mit 8-24 Kohlenstoffatomen, R³ für eine Alkylengruppe mit 1-18 Kohlenstoffatomen, Y für eine OH-Gruppe oder für Wasserstoff und X für ein einwertiges, organisches oder anorganisches Anion steht
und/oder
- einen Polyoxyalkylen-Ether eines Polyglycerin-Fettsäureesters (B)
enthält.

2. Zubereitung nach Anspruch 1, dadurch gekennzeichnet, daß eine Phase Wasser oder eine Emulsion ist, die bis zu 5 Gew.-% Öl, bezogen auf die Komponenten Wasser und Öl, enthält.

3. Zubereitung nach Anspruch 1 oder 2, dadurch gekennzeichnet, daß eine Phase eine Öl-Phase ist, die bis zu 20 Gew.-% Wasser, bezogen auf die Komponenten Öl und Wasser, enthält.

4. Zubereitungen nach einem der Ansprüche 1 bis 3, dadurch gekennzeichnet, daß die beiden Phasen in einem Massen-Verhältnis von 1:1 bis 5:1 vorliegen.

5. Zubereitung nach einem der Ansprüche 1 bis 4, dadurch gekennzeichnet, daß (A) eine Verbindung der Formel (I) ist, in der R eine Alkyl- oder Alkenylgruppe mit 8-24 Kohlenstoffatomen, R¹ und R² Methylgruppen, und R³ eine Alkylengruppe mit 1-4 Kohlenstoffatomen, Y eine Hydroxygruppe und X ein Halogenidion ist.

6. Zubereitung nach einem der Ansprüche 1 bis 5, dadurch gekennzeichnet, daß (B) - 2 bis 20 Oxyalkylen-, insbesondere Oxyethylen-, Einheiten,
- 2 bis 10 Glycerin-Einheiten sowie
- 1 oder 2 Fettsäureeinheiten mit 8 bis 24 Kohlenstoffatomen aufweist.

7. Zubereitung nach einem der Ansprüche 1 bis 6, dadurch gekennzeichnet, daß das Panthenolderivat (A) in einer Menge von 0,05-1,0 Gew.-%, insbesondere 0,05-0,2 Gew-%, bezogen auf die gesamte Zubereitung, enthalten ist.

8. Zubereitung nach einem der Ansprüche 1 bis 7, dadurch gekennzeichnet, daß die Komponente (B) in einer Menge von 0,05-1 Gew.-%, insbesondere 0,1-0,5 Gew-%, bezogen auf die gesamte Zubereitung, enthalten ist.

9. Zubereitung nach einem der Ansprüche 1 bis 8, dadurch gekennzeichnet, daß jede der beiden Phasen zumindest einen kosmetischen Wirkstoff enthält.

10. Verfahren zur Behandlung von Haaren, dadurch gekennzeichnet, daß eine Zubereitung nach einem der Ansprüche 1 bis 9 auf das Haar aufgebracht wird und dort verbleibt.

11. Verfahren zur Behandlung von Haaren, dadurch gekennzeichnet, daß eine Zubereitung nach einem der Ansprüche 1 bis 9 auf das Haar aufgebracht wird und nach einer Einwirkzeit wieder ausgespült wird.

## Claims

1. A preparation in the form of a 2-phase system mixable in a short time by mechanical action for the treatment of keratin structures, more particularly human hair and human skin, characterized in that it contains
- a cationically derivatized panthenol (A) corresponding to general formula (I): 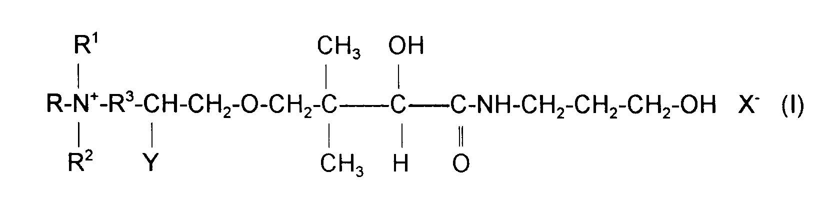 in which R, R¹ and R² independently of one another represent an alkyl group containing 1 to 24 carbon atoms or an alkenyl group containing 8 to 24 carbon atoms, R³ is an alkylene group containing 1 to 18 carbon atoms, Y is an OH group or hydrogen and X is a monovalent organic or inorganic anion
and/or
- a polyoxyalkylene ether of a polyglycerol fatty acid ester (B).

2. A preparation as claimed in claim 1, characterized in that one phase is water or an emulsion which contains up to 5% by weight of oil, based on the components water and oil.

3. A preparation as claimed in claim 1 or 2, characterized in that one phase is an oil phase which contains up to 20% by weight of water, based on the components oil and water.

4. Preparations as claimed in any of claims 1 to 3, characterized in that the two phases are present in a ratio by weight of 1:1 to 5:1.

5. A preparation as claimed in any of claims 1 to 4, characterized in that (A) is a compound corresponding to formula (I) in which R is an alkyl or alkenyl group containing 8 to 24 carbon atoms, R¹ and R² are methyl groups and R³ is an alkylene group containing 1 to 4 carbon atoms, Y is a hydroxy group and X is a halide ion.

6. A preparation as claimed in any of claims 1 to 5, characterized in that (B) contains
- 2 to 20 oxyalkylene units, more especially oxyethylene units,
- 2 to 10 glycerol units and
- 1 or 2 fatty acid units containing 8 to 24 carbon atoms.

7. A preparation as claimed in any of claims 1 to 6, characterized in that the panthenol derivative (A) is present in a quantity of 0.05 to 1.0% by weight and, more particularly, 0.05 to 0.2% by weight, based on the preparation as a whole.

8. A preparation as claimed in any of claims 1 to 7, characterized in that component (B) is present in a quantity of 0.05 to 1% by weight and, more particularly, 0.1 to 0.5% by weight, based on the preparation as a whole.

9. A preparation as claimed in any of claims 1 to 8, characterized in that each of the two phases contains at least one cosmetic active substance.

10. A hair treatment process, characterized in that the preparation claimed in any of claims 1 to 9 is applied to and left on the hair.

11. A hair treatment process, characterized in that the preparation claimed in any of claims 1 to 9 is applied to the hair and, after a contact time, is rinsed off.

## Revendications

1. Composition présente sous forme d'un système à deux phases, mélangeable en peu de temps par action mécanique, pour le traitement des édifices kératinisés, en particulier les cheveux humains et la peau humaine, caractérisée en ce qu'elle contient :
- un panthénol dérivatisé cationique (A) de formule générale (I) : 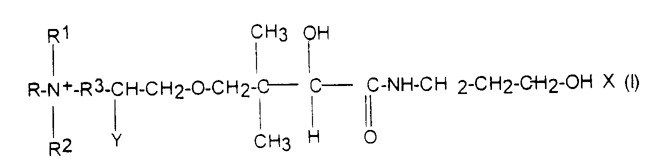 dans laquelle R, R¹ et R² représentent, indépendamment l'un de l'autre, un radical alcoyle ayant 1 à 24 atomes de carbone ou un radical alcoylène ayant 8 à 24 atomes de carbone, R³ un radical alcoylène ayant 1 à 18 atomes de carbone, Y un radical OH ou un atome d'hydrogène et X un anion monovalent organique ou inorganique, et/ou
- un éther de polyoxyalcoylène d'un ester polyglycérinique d'acide gras. (B).

2. Composition suivant la revendication 1, caractérisée en ce qu'une phase est de l'eau ou une émulsion, qui contient jusqu'à 5% en poids d'huile, sur base des composants eau et huile.

3. Composition suivant la revendication 1 ou 2, caractérisée en ce qu'une phase est une phase huileuse, qui contient jusqu'à 20% en poids d'eau, sur base des composants huile et eau.

4. Composition suivant l'une quelconque des revendications 1 à 3, caractérisée en ce que les deux phases sont présentes dans un rapport massique allant de 1:1 à 5:1.

5. Composition suivant l'une quelconque des revendications 1 à 4, caractérisée en ce que (A) est un composé de formule (I), dans lequel R est un radical alcoyle ou alcoylène ayant 8 à 24 atomes de carbone, R¹ et R² sont des radicaux méthyle et R³ est un radical alcoylène ayant 1 à 4 atomes de carbone, Y est un radical hydroxyle et X est un ion halogène.

6. Composition suivant l'une quelconque des revendications 1 à 5, caractérisée en ce que (B) présente :
- 2 à 20 unités oxyalcoylène, en particulier oxyéthylène ;
- 2 à 10 unités glycérine, ainsi que
- 1 ou 2 unités acide gras ayant 8 à 24 atomes de carbone.

7. Composition suivant l'une quelconque des revendications 1 à 6, caractérisée en ce que le dérivé du panthénol (A) est présent en une quantité allant de 0,05 à 1,0% en poids, en particulier de 0,05 à 0,2% en poids, sur base de la composition totale.

8. Composition suivant l'une quelconque des revendications 1 à 7, caractérisée en ce que le composé (B) est présent en une quantité allant de 0,05 à 1% en poids, en particulier de 0,1 à 0,5% en poids, sur base de la composition totale.

9. Composition suivant l'une quelconque des revendications 1 à 8, caractérisée en ce que chacune des deux phases contient au moins un agent actif cosmétique.

10. Procédé de traitement des cheveux, caractérisé en ce qu'une composition suivant l'une quelconque des revendications 1 à 9 est appliquée sur les cheveux et y reste.

11. Procédé de traitement des cheveux, caractérisé en ce qu'une composition suivant l'une quelconque des revendications 1 à 9 est appliquée sur les cheveux et est éliminée par rinçage après une période d'action.
